# EUROPEAN PATENT APPLICATION

(11) **EP 4 789 676 A1**
(43) Date of publication of application: **12.08.2026**
(21) Application number: 25382086.4
(22) Date of filing: 05.02.2025
(51) Int. Cl.: A61K 35/745, A61K 35/747, A23L 33/135, A61P 17/00

(54) **PROBIOTIC COMPOSITIONS FOR THE TREATMENT OF ROSACEA**

(71) Applicant: Bioithas S.L., 03430 Onil Alicante (ES)
(72) Inventor: NAVARRO LÓPEZ, Vicente, 03008 Alicante (ES); NAVARRO MORATALLA, Laura, 03008 Alicante (ES)
(74) Representative: Hoffmann Eitle

(57) **Abstract**

The present invention relates to probiotic composition comprising a combination of *L. rhamnosus* and *B*. *longum* strains, specifically *L. rhamnosus* CECT 30579 and B. *longum* CECT 30615 and their use in the treatment of Rosacea.

## Description

### Technical field

The present invention relates to probiotic composition comprising a combination of *L*. *rhamnosus* and B. *longum* strains, specifically *L. rhamnosus* CECT 30579 and B. *longum* CECT 30615 and their use in the treatment of Rosacea.

### Background of the invention

Rosacea is a chronic skin disease affecting approximately 5.5% of general population, mainly patients between 45 and 60 years old, regardless of the sex (Gether et al., 2018). Rosacea mainly appears on the cheeks, nose, chin, and forehead, with alternating periods of remission and aggravation. Cutaneous symptoms comprise persistent erythema, papules, pustules, telangiectasia, flushing, sebaceous glands hypertrophy, and fibrosis, characteristically referred to as phyma (van Zuuren, 2017).

Given the chronic nature of Rosacea, there is considerable evidence to support its association with various systemic comorbidities which could indicate a systemic inflammatory state. In this context, Rosacea is linked with hypertension, dyslipidemia, atherosclerosis, other cardiovascular diseases, gastrointestinal diseases (this issue will be further developed), migraine, anxiety, depression, and even several malignancies (Morss-Walton and McGee, 2021).

The variable clinical spectrum mentioned above is illustrated by the multifactorial origin of Rosacea. Therefore, several phenotypes of this diseases are recognized as part of an ongoing inflammatory process. On this basis, with current knowledge of its pathophysiology, the national Rosacea society (NRS) published in 2017 a new standard classification system (Gallo et al., 2018). The previous 2002 classification yet identified the main signs and symptoms of Rosacea and categorized them into four subtypes: erythematotelangiectatic (ETR), papulopustular (PPR), phymatous, and ocular Rosacea, being ETR and PPR the top phenotypes diagnosed (Barakji et al., 2022). The problem with this system was that it did not consider the frequent coexistence of phenotypes and progression from one subtype to another. The updated 2017 classification requires at least one diagnostic criteria (fixed centrofacial erythema in a characteristic pattern that may periodically intensify, phymatous changes) or two major or primary phenotypes (papules and pustules, flushing, telangiectasia, ocular manifestations). In addition, secondary signs, and symptoms (burning, itching, edema, dryness) may also appear in conjunction with one or more diagnostic or primary phenotypes. The global Rosacea consensus panel (ROSCO) recommendations supported in 2019 this NRS classification (Schaller et al., 2020). Importantly, Rosacea can progress not only to additional phenotypes, but also in severity.

The treatment of Rosacea should be based on these phenotypes (according to new NRS classification) (Gallo et al., 2018) and severity. Thus, there are several first-line treatments and in some cases a maintenance schedule could be justified depending on the clinical evolution and background of the patient. Usually in moderate to severe cases a combined approach of oral and topical therapy is required. Moreover, there are a variety of important general instructions for the management of several manifestations of Rosacea, including non-aggressive hygiene measures, frequent use of moisturizers and photoprotectors, and elimination or mitigation of recognized aggravating factors (e.g., heat and some foods) (Schaller et al., 2017a; Salleras et al., 2019; Clanner-Engelshofen et al., 2022).

However, Rosacea is a difficult disease to keep under control. Some studies have shown that approximately 80% of Rosacea patients consider their facial erythema to be unpredictable (Dirschka et al., 2015). In many cases the Rosacea patient presents a history of therapeutic failure or insufficient results, but it should always be insisted for 6 - 8 weeks until an exacerbation treatment was considered ineffective (Schaller et al., 2017a; Salleras et al., 2019; Clanner-Engelshofen et al., 2022). At present, not all patients achieve complete resolution of symptoms. Therefore, there is still a need to find more effective treatments.

A main reason for seeking new therapeutic approaches in Rosacea is that its symptomatology can have an emotional impact and even an impact on social relationships resulting in stigmatization of the subject inflicted by this disease. It is therefore important to offer novel effective and simple treatment options.

The term probiotic has been defined as "living microorganisms which, when consumed in adequate amounts, confer a health effect on the host" (World Health Organization and Food and Agriculture Organization of the United Nations, 2001). During the past few decades, there has been renewed interest in probiotics not only regarding digestive health, but also in the management of inflammatory diseases.

Oral probiotics have been shown to improve insulin sensitivity in animal models as well as regulate the release of inflammatory cytokines in the skin through their interaction with gut-associated lymphoid tissue (Hacini-Rachinel et al, 2009). In fact, the gut-brain-skin axis suggests a mechanism that links gastrointestinal health, influenced by interactions with oral probiotics, to the health and well-being of the skin (Bowe W.P. et al, 2011).

Several strains of *Lacticaseibacillus* have also been demonstrated to have anti-inflammatory properties.

Rosacea is a skin condition in which the skin barrier is impaired, and symptoms improve when the skin barrier is strengthened. Oral ingestion of certain probiotic strains has been shown to improve the skin barrier and affect skin hydration and transepidermal water loss. In fact, Gueniche et al. (2014) studied the effects of oral supplementation with *L*. *paracasei* NCC2461 versus placebo for healthy female volunteers in a randomized placebo-controlled clinical trial. A capsaicin test was used to monitor skin sensitivity, while trans-epidermal water loss and dermatological assessments were utilized to measure skin barrier function. Both skin sensitivity and skin barrier function improved in the probiotic group. The probiotic group also showed increases in the serum concentration of TGF-beta after 29 days compared to no increase in the placebo group. TGF-beta has been shown to play a significant role in skin integrity.

Thus, overall, probiotics modulate the development of the immune system, often shifting the immune response toward regulatory and anti-inflammatory conditions. This ability of probiotics to modify chronic inflammatory states suggests that probiotics may have a role in treating chronic inflammatory conditions, ranging from inflammatory bowel disease to reactive airway disease to acne, Rosacea, atopic dermatitis, and photoaging (Benyacoub J. et al, 2014).

However, there is still lacking an effective probiotic composition suitable for the successful treatment and prevention of Rosacea. The inventors have therefore set out to solve this problem by developing new probiotic compositions that are suitable for treating Rosacea in a simple and effective manner.

### Summary of the Invention

In one aspect the present invention relates to a probiotic composition comprising:
a*. Lacticaseibacillus rhamnosus (L. rhamnosus)* strain deposited under the Budapest treaty in the Spanish Type culture Collection (CECT) under accession number CECT 30579; and
b*. Bifidobacterium longum (B. longum)* strain deposited under the Budapest treaty in the Spanish Type culture Collection (CECT) under accession number CECT 30615.

In one embodiment the probiotic composition comprises of between 10⁶ and 10¹² cfu, preferably between 10⁹ and 10¹¹ cfu of *L. rhamnosus* CECT 30579, and between 10⁶ and 10¹² cfu, preferably between 10⁹and 10¹¹ cfu of B. *longum* CECT 30615.

In one embodiment the probiotic composition comprises a total amount of between 10⁶ and 10¹² cfu, preferably between 10⁹ and 10¹¹ cfu of *L. rhamnosus* CECT 30579 and B. *longum* CECT 30615.

In one embodiment the probiotic composition is in the form of a lyophilized (freeze-dried) powder.

In one embodiment said probiotic composition is formulated for administration in liquid form or in solid form, preferably wherein said composition is formulated for oral administration.

In one embodiment said probiotic composition is a pharmaceutical composition comprising pharmaceutically acceptable excipients.

In one embodiment said probiotic composition is a nutritional composition or nutritional supplement.

In a preferred embodiment said pharmaceutical composition, nutritional composition or nutritional supplement composition is in the form of a formulation selected from the group consisting of tablets, lozenges, sweets, chewable tablets, chewing gum, capsules, sachets, powders, granules, coated particles or coated tablets, tablets and gastro-resistant tablets and capsules and dispersible strips and films, or in the form of a liquid formulation selected from the group consisting of oral solutions, suspensions, emulsions and syrups.

In a further aspect the present invention relates to the probiotic composition as disclosed herein for use in therapy.

In one embodiment the present invention relates to the probiotic composition as disclosed herein for use in a method of easing, reducing, treating and/or preventing Rosacea in a subject, preferably a human subject, in need thereof.

In one embodiment said method further comprises orally administering to said subject an effective amount of the composition thereby treating Rosacea.

In one embodiment at least one additional compound is administered in an effective amount for treating Rosacea to said subject. In a preferred embodiment said additional compound acts as an adjuvant.

In a further aspect the present invention relates to a probiotic composition comprising *Lacticaseibacillus rhamnosus* strain deposited under the Budapest treaty in the Spanish Type culture Collection (CECT) under accession number CECT 30579 for use in a method of easing, reducing, treating and/or preventing Rosacea in a subject, preferably a human subject, in need thereof.

In another aspect the present invention relates to a probiotic composition comprising B. *longum* strain deposited under the Budapest treaty in the Spanish Type culture Collection (CECT) under accession number CECT 30615 for use in a method of easing, reducing, treating and/or preventing Rosacea in a subject, preferably a human subject, in need thereof.

In one embodiment of any of the above aspects the probiotic composition is administered orally to the subject in need thereof.

### Brief description of the figures

**Figure 1****.** Pro-inflammatory cytokine production in response to *L. rhamnosus* CECT 30579. Data analysed with Student's t-tests. * = p < 0.05, ** = p < 0.01, and *** = p < 0.001. A) TNF-α; B) IL-6.
**Figure 2****.** Anti-inflammatory cytokine (TGF-β) production in response to *L. rhamnosus* CECT 30579. Data analysed with Student's t-tests. * = p < 0.05, ** = p < 0.01, and *** = p < 0.001.
**Figure 3****.** Pro-inflammatory cytokine production in response to *B*. *longum* CECT 30615. Data analyzed with Student's t-tests. * = p < 0.05, ** = p < 0.01 and *** = p < 0.001.

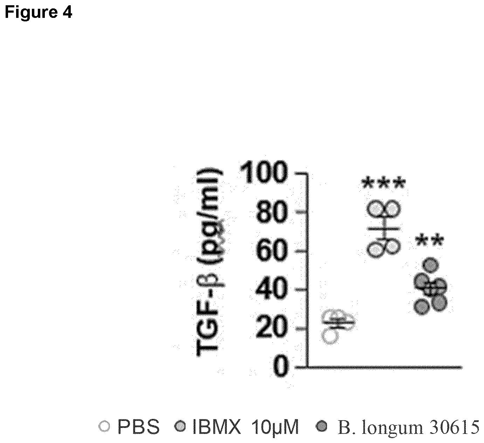

**Figure 4****.** Anti-inflammatory cytokine (TGF-β) production in response to *B*. *longum* CECT 30615. Data analyzed with Student's t-tests. * = p < 0.05, ** = p < 0.01 and *** = p < 0.001.

### Detailed Description of the invention

As described above, Rosacea is a difficult disease to keep under control and in many cases the Rosacea patient presents a history of therapeutic failure or insufficient results. Numerous studies have highlighted a negative impact on the health-related quality of life in Rosacea patients and at present and with the currently available treatment options not all patients achieve complete resolution of symptoms and there is still a need for further effective treatment options.

The inventors have therefore set out to identify novel compositions that provide for a simple and efficacious treatment of Rosacea.

As described in Example 1 different bacterial strains from the species *Lacticaseibacillus rhamnosus* (formerly *Lactobacillus rhamnosus*), were selected and their anti-inflammatory properties tested by comparing their effect on certain blood markers (IL-10, LPS or bacterial DNA). *L. rhamnosus* CECT 30579 strain showed the best results in blood markers. In addition, the same strains were tested in a small study including five patients per probiotic strain, also demonstrating that *L. rhamnosus* CECT 30579 was the one with the best clinical results.

Furthermore, bacterial strains from the species *Bifidobacterium longum* were tested in an *in vitro* study and the strain *B*. *longum* CECT 30615 showed greater systemic anti-inflammatory properties with a greater increase in IL-10 levels than the other *B*. *longum* strains. These strains were therefore chosen for further testing.

The inventors could furthermore show the immunomodulatory capacity of *L. rhamnosus* CECT 30579 and *B*. *longum* CECT 30615 as further described in Example 2.

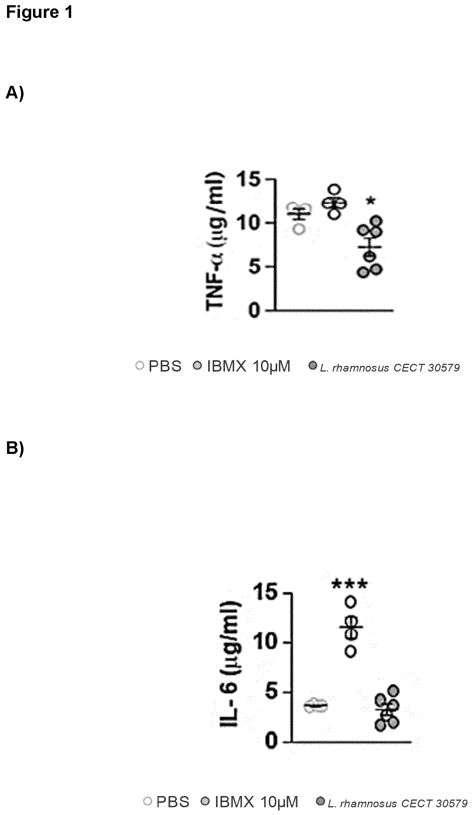

In summary, PBMC cell cultures treated with *L. rhamnosus* CECT 30579 showed a significant decrease in TNF-alpha, a pro-inflammatory cytokine (Fig.1A). On the other hand, exposure to *L. rhamnosus* CECT 30579 to PBMC cultures did not increase IL-6 production, in contrast to the response generated by LPS (Fig.1B). In terms of anti-inflammatory cytokine production, both treatments (LPS and *L. rhamnosus* 30579) significantly increased TGF-β production, although the increase was less pronounced in the case of the bacteria (Fig.1C).

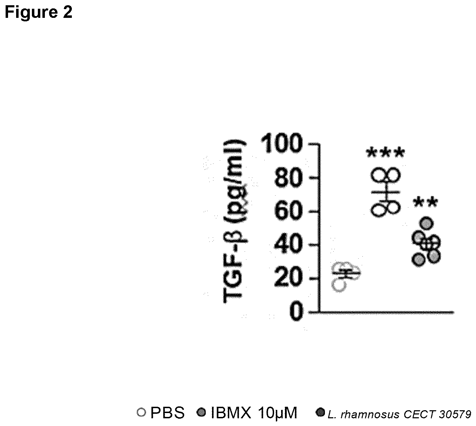

PBMC cell cultures treated with *B*. *longum* CECT 30615 as well as the cultures exposed to LPS, showed a significant increase in IFN-γ, a pro-inflammatory cytokine involved in macrophage activation, and MCP-1, which is involved in monocyte migration. Regarding anti-inflammatory cytokine production, both treatments (LPS and *B*. *longum* CECT 30615) significantly increased TGF-β production (Fig.2).

The results are in line with previous work showing that some strains enhance the anti-inflammatory response and also reduce the levels of pro-inflammatory cytokines (TNF-α expression) in PBMC cultures pre-stimulated with LPS (Barigela et al., 2021). The inventors could show the anti-inflammatory potential of *L. rhamnosus* CECT 30579 in view of the lower induction of IL-6 production than that elicited by LPS, together with the increase of the anti-inflammatory cytokine TGF-β.

When evaluating the effect of *B*. *longum* CECT 30615 on other cytokines, they demonstrated an effect on the production of pro-inflammatory cytokines such as interferon gamma (IFN-γ) with both LPS and probiotic, although there is a lower pro-inflammatory induction of MCP-1 with probiotic than with LPS. Finally, both LPS and *B*. *longum* CECT 30615 have an induction in the production of anti-inflammatory cytokines with a significant increase in TGF-β production.

These different results can be attributed to the existence of differences between the two probiotic strains tested, and their possible action through different mechanisms making them a suitable combination for a more effective treatment of Rosacea.

Following these results *L. rhamnosus* CECT 30579 and *B*. *longum* CECT 30615 were tested in a small pilot study involving 20 subjects that were separated into groups of 5 patients that were either administered *L. rhamnosus* CECT 30579 or *B*. *longum* CECT 30615 only, or a mixture of *L. rhamnosus* CECT 30579 and *B. longum* CECT 30615. In the group receiving only probiotic *L. rhamnosus* CECT 30579 one patient of five (20%) improved in at least one category in the IgA score. In the group receiving only probiotic *B. longum* CECT 30615 two patients of five (40%) improved in at least one category in the IgA score. Surprisingly, when the mixture of the two probiotics was used 4 of the 5 patients (80%) improved in some of the scales.

This data suggested a synergistic effect of the two strains when administered together and a double-blind confirmatory pilot study using the composition comprising *L*. *rhamnosus* CECT 30579 and *B*. *longum* CECT 30615 was performed.

As described in more detail in Example 3, as a next step a randomized, double-blind, placebo-controlled clinical trial to evaluate the effect of the composition comprising *L*. *rhamnosus* CECT 30579 and *B*. *longum* CECT 30615 on the evolution and treatment of Rosacea was performed with 60 patients.

An intermediate analysis of the most relevant outcomes including 50 patients (the analysis of the remaining 10 is pending) and comparing data between the initial visit (visit 1-baseline) and the end-of-treatment visit (visit 3 -12 weeks visit) has shown that according to the Investigator's Global Assessment (IGA) index score in the probiotic group, the number and percentage of cases responding to the treatment according to the IGA score were 17/25 (68%), while in the placebo group, it was 12/25 (48%). Therefore, already in this first data set a 20% improvement in the subjects taking the probiotic composition comprising *L. rhamnosus* CECT 30579 and *B*. *longum* CECT 30615 of present invention was observed and is clinically significant.

Based on these results, the inventors conclude that present invention provides a novel and improved treatment of Rosacea with a probiotic composition comprising the combination *L. rhamnosus* CECT 30579 and *B*. *longum* CECT 30615.

Furthermore, and as illustrated in examples 1 and 2, each of the strains *L. rhamnosus* CECT 30579 and *B. longum* CECT 30615 were also evaluated separately for their immunomodulatory activity and the treatment of Rosacea. In the small pilot study performed with five patients in each of the three treatment branches (*L. rhamnosus* CECT 30579 only, *B. longum* CECT 30615 only, and combination of *L. rhamnosus* CECT 30579 and *B. longum* CECT 30615) showed that the effect is significantly improved when both strains are used in combination compared with the effect of each of the strains separately. However, each strain separately also shows, even if less pronounced, an effect on the treatment of Rosacea.

Thus, in a first aspect the present invention relates to a probiotic composition comprising:
a. *Lacticaseibacillus rhamnosus* strain deposited under the Budapest treaty in the Spanish Type culture Collection (CECT) under accession number CECT 30579; and
b. *B. longum* strain deposited under the Budapest treaty in the Spanish Type culture Collection (CECT) under accession number CECT 30615.

In a further aspect the present invention relates to the probiotic composition as disclosed herein for use in therapy.

In one embodiment the present invention relates to the probiotic composition for use in in a method of easing, reducing, treating and/or preventing Rosacea in a subject, preferably a human subject, in need thereof.

In one embodiment said method further comprises orally administering to said subject an effective amount of the composition thereby treating Rosacea.

In one embodiment at least one additional compound is administered in an effective amount for treating Rosacea to said subject. In a preferred embodiment said additional compound acts as an adjuvant.

In a further aspect the present invention relates to a probiotic composition comprising *Lacticaseibacillus rhamnosus* strain deposited under the Budapest treaty in the Spanish Type culture Collection (CECT) under accession number CECT 30579 for use in a method of easing, reducing, treating and/or preventing Rosacea in a subject, preferably a human subject, in need thereof.

In another aspect the present invention relates to a probiotic composition comprising *B. longum* strain deposited under the Budapest treaty in the Spanish Type culture Collection (CECT) under accession number CECT 30615 for use in a method of easing, reducing, treating and/or preventing Rosacea in a subject, preferably a human subject, in need thereof.

In a further aspect the present invention relates to a probiotic composition comprising:
a. at least one *L. rhamnosus* strain; and
b. at least one B. *longum* strain.

In the present invention the term "probiotic composition", as used in any of the embodiment of present invention, is understood as a composition comprising microorganisms which, when ingested, interact with the individual's metabolism and produce a beneficial effect in it. In the present invention, the probiotic composition comprises a mixture of at least one strain of *Lacticaseibacillus* and at least one strain of *Bifidobacterium.*

The preferred Lacticaseibacillus is *L. rhamnosus. L. rhamnosus* is a bacterium commonly used as a probiotic, found mostly in yogurt and other dairy products, including infant formula. *L. rhamnosus* is classified as follows: Domain: Bacteria, Phylum: Bacillota, Class: Bacilli, Order: Lactobacillales, Family: Lactobacillaceae, Genus: *Lacticaseibacillus,* Species: *L. rhamnosus.*

The most preferred *Lacticaseibacillus* is *L. rhamnosus* CECT 30579.

The preferred *Bifidobacterium* is *B.longum. Bifidobacterium* is a genus of gram-positive, nonmotile, often branched anaerobic bacteria that are ubiquitous inhabitants of the gastrointestinal tract. The scientific classification of *B.longum* is: Domain: Bacteria, Phylum: Actinomycetota, Class: Actinomycetia Order: Bifidobacteriales, Family: Bifidobacteriaceae, Genus: *Bifidobacterium,* Species: *B. longum.*

The most preferred Bifidobacterium is *B*. *longum* CECT 30615.

In the present invention the term "subject" is equivalent to the term "individual", so both terms can be used interchangeably herein. "Subject" means, in addition to any individual, any animal belonging to any species. Examples of subjects include, but are not limited to, animals of commercial interest such as birds (hens, ostriches, chicks, geese, partridges, etc.), rabbits, hares, pets (dogs, cats, etc.), sheep, goat cattle (goats, etc.), swine (boars, pigs, etc.), equine livestock (horses, ponies, etc.), cattle (bulls, cows, oxen, etc.); animals of hunting interest, such as stags, deer, reindeer, etc.; and humans. However, in a particular embodiment, the subject is a mammal, particularly the mammal is a human being of any race, sex or age.

In the present invention the term "prevention" means to avoid occurrence of the disease or pathological condition in an individual, particularly when the individual has predisposition for the pathological condition but has not yet been diagnosed. In a preferred embodiment of the present invention, the disease or pathological condition is "Rosacea".

In the present invention, the term "treat" or "treatment" comprises inhibiting the disease or pathological condition, i.e., stopping its development; relieving the disease or pathological condition, i.e., causing regression of the disease or pathological condition; relieving or reducing at least one of the symptoms or signs of the disease or pathological condition; and/or stabilizing the disease or pathological condition in an individual. In a preferred embodiment of the present invention, the disease or pathological condition is "Rosacea".

In the context of the present invention, the term "Rosacea" refers to the commonly known chronic skin disease, usually affecting the face. It causes redness, erythema, pustules or papules, swelling and dilated small, superficial blood vessels. Often the nose, cheeks, forehead and chin are the most affected areas. It is to be understood that the term "Rosacea" as used herein is to be differentiated from the term acne and its most common form the "acne vulgaris" as well as its milder form referred to as "comedones", which are non-inflammatory. The most common factors that caused acne vulgaris are hormones, increased sebum production, bacteria (*Cutibacterium Acnes*), systemic and local inflammation and changes inside of the hair follicle. Whilst Rosacea can look similar to acne vulgaris, and the two are sometimes confused for one another, Rosacea affects millions of people, most of whom are over the age of 30, whereas acne vulgaris is a more common problem in adolescents. Its exact cause is not fully known, but is thought to involve genetic, immunological, environmental and microbial factors. One of the factors that has been investigated in Rosacea is the abnormal proliferation of the *Demodex folliculorum* mite. This is a microscopic mite that naturally inhabits the hair follicles and sebaceous glands of human skin. However, in people with Rosacea, a significant increase in the number of these mites has been observed.

It is noted that the present invention also contemplates those microorganisms or bacteria derived from the microorganism *L. rhamnosus* CECT 30579 which retains the ability to reduce and/or improve the evolution of Rosacea in an individual in need thereof of said *L. rhamnosus* CECT 30579.

The present invention also contemplates those microorganisms or bacteria derived from the microorganism *B. longum* CECT 30615 which retains the ability to reduce and/or improve the evolution of Rosacea in an individual in need thereof of said *B. longum* CECT 30615.

Such derived bacteria may be part of, or totally or partially replacing, any of microorganisms *L. rhamnosus* CECT 30579 and/or *B. longum* CECT 30615, in any of the above probiotic compositions of the invention as long as they retain the ability to reduce and/or improve the evolution of Rosacea in an individual in need thereof.

In the context of the present invention "retains the ability to reduce and/or improve the evolution of Rosacea in an individual in need thereof" shall be understood as a significant improvement, in comparison to a subject treated with a placebo according to the Investigator's Global Assessment (IGA) index score. The IGA index measures the severity of Rosacea, where the score range is from 0 to 6 according to the severity of the disease as follows: 0=clear; 1=minimum; 2=mild; 3=mild-moderate; 4=moderate; 5=moderate-severe; 6=severe.

It will be considered a treatment response in IGA scale when the participant improves by at least 2 categories in this index and the final score is 0-1 points (clear and almost clear).

Examples of strains or microorganisms derived from strains comprised within any of the above-mentioned probiotic compositions of the invention may be mutants and genetically modified organisms which show variations in their genome compared to the genome of the strains of the invention from which they derived, but which do not affect the ability of strains to reduce and/or improve the evolution of Rosacea in the individual. Strains derived from *L. rhamnosus* CECT 30579 *and B. longum* CECT 30615, can be naturally or intentionally produced by mutagenesis methods known in the art such as for example, but not limited to, the growth of the parent strain in the presence of mutagenic agents or stressors or by genetic engineering directed to the modification, deletion and/or insertion of specific genes. Thus, as indicated above, the present invention also contemplates genetically modified organisms derived from *L. rhamnosus* CECT 30579 and *B. longum* CECT 30615, that retain the ability to reduce and/or improve the evolution of Rosacea in an individual and, therefore, to be used in the treatment of Rosacea.

Furthermore, the present invention, also contemplates cellular components, metabolites and molecules secreted by the *L. rhamnosus* CECT 30579 and *B. longum* CECT 30615 strains as well as compositions comprising said components and uses thereof for the treatment and/or prevention of Rosacea. The cellular components of bacteria could include components of the cell wall (such as, but not limited to, peptidoglycan), nucleic acids, membrane components and other, such as proteins, lipids and carbohydrates and combinations thereof (such as lipoproteins, glycolipids or glycoproteins). Metabolites include any molecule produced or modified by the bacterium or microalgae as a result of its metabolic activity during growth, its use in technological processes or during storage of the product (first probiotic composition of the invention). Examples of these metabolites include, but are not limited to, organic and inorganic acids, proteins, peptides, amino acids, enzymes, lipids, carbohydrates, lipoproteins, glycolipids, glycoproteins, vitamins, salts, minerals or nucleic acids. Secreted molecules include any molecule secreted or released to the outside by the bacterium during growth, its use in technological processes (for example, food processing or drugs) or during storage of the product. Examples of these molecules include, but are not limited to, organic and inorganic acids, proteins, peptides, amino acids, enzymes, lipids, carbohydrates, lipoproteins, glycolipids, glycoproteins, vitamins, salts, minerals or nucleic acids.

As understood by those skilled in the art, any of the probiotic compositions of the invention may be formulated for pharmaceutical administration, i.e., forming part of pharmaceutical products to be administered to the subject (either orally, topically, etc., preferably orally), and/or for food administration, i.e. forming part of the foods consumed in the subject's diet, thus being administered orally.

Therefore, in a particular embodiment, any of the compositions or probiotic compositions of the invention can be a pharmaceutical composition (from hereinafter pharmaceutical composition of the invention) and/or a nutritional composition (from hereinafter nutritional composition of the invention). Such pharmaceutical compositions of the invention can thus be used in the treatment and/or prevention of Rosacea.

The pharmaceutical compositions of the invention thus comprise *L. rhamnosus* CECT 30579 and/or *B. longum* CECT 30615 (or strains derived therefrom as defined above) at any concentration and may additionally comprise one or more components or compounds having any biological, pharmacological and/or veterinary useful activity in the prevention and/or treatment of Rosacea. Such pharmaceutical compositions may further comprise one or more components which, upon administration to a subject, may further increase, enhance and/or promote the activity of the strain or strains included in any of the probiotic compositions of the invention. As understood by one skilled in the art, the additional components or compounds must be compatible with the strains of any of the probiotic compositions of the invention. In the context of the present invention, the term "pharmaceutical composition" also encompasses veterinary compositions.

In a particular embodiment, the pharmaceutical composition of the invention further comprises a pharmaceutically acceptable carrier and/or excipient.

The term "excipient" refers to a substance that helps the absorption of any components or compounds of any of the probiotic compositions of the invention, namely, of strains of the invention, or stabilizes the components or compounds and/or assists the preparation of the pharmaceutical composition in the sense of giving it consistency or flavors to make it more pleasant. Thus, the excipients may have the function, by way of example but not limited thereto, of binding the components (for example, starches, sugars or cellulose), sweetening, coloring, protecting the active ingredient (for example, to insulate it from air and/or moisture), filling a pill, capsule or any other presentation or a disintegrating function to facilitate dissolution of the components, without excluding other excipients not listed in this paragraph. Therefore, the term "excipient" is defined as that material that included in the galenic forms, is added to the active ingredients or their associations to enable their preparation and stability, modify their organoleptic properties or determine the physico-chemical properties of the pharmaceutical composition and its bioavailability. The "pharmaceutically acceptable" excipient must allow the activity of components or compounds of the pharmaceutical composition, that is, be compatible with the strain or strains of the invention.

The "galenic form" or "pharmaceutic form" is the configuration to which the active ingredients and excipients are adapted to provide the pharmaceutical composition or drug of the invention. It is defined by the combination of the form in which the pharmaceutical composition is presented by the manufacturer and the form in which it is administered.

The "vehicle" or "carrier" is particularly an inert substance. Carrier functions are to facilitate the incorporation of other components or compounds, allow better dosage and administration and/or give consistency and form to the pharmaceutical composition. Therefore, the carrier is a substance used in the drug to dilute any of the components or compounds of the pharmaceutical composition of the present invention to a given volume or weight; or that even without diluting these components or compounds, it is able to allow better dosage and administration and/or give consistency and form to the drug. When the presentation is liquid, the pharmaceutically acceptable carrier is the diluent. The carrier can be natural or unnatural. Examples of pharmaceutically acceptable carriers include, without being limited thereto, water, salt solutions, alcohol, vegetable oils, polyethylene glycols, gelatin, lactose, starch, amylose, magnesium stearate, talc, surfactants, silicic acid, viscous paraffin, perfume oil, monoglycerides and diglycerides of fatty acids, fatty acid esters petroetrals, hydroxymethylcellulose, polyvinylpyrrolidone and the like.

Furthermore, the excipient and the carrier must be pharmacologically acceptable, i.e., the excipient and the carrier are permitted and evaluated so as not to cause damage to the subject to whom it is administered.

In each case the presentation of the pharmaceutical composition will be adapted to the type of administration used. Thus, the composition may be presented in the form of solutions or any other form of clinically permissible administration and in a therapeutically effective amount. The pharmaceutical composition can be thus formulated into solid, semisolid or liquid preparations, such as tablets, capsules, powders (such as those derived from lyophilization (freeze-drying) or air-drying), granules, solutions, suppositories, gels or microspheres. In a particular embodiment, the pharmaceutical composition is formulated for administration in liquid form or in solid form. In an embodiment, the composition is in form of gelatin capsules.

In another particular embodiment, the solid formulation is selected from the group consisting of tablets, lozenges, sweets, chewable tablets, chewing gums, capsules, sachets, powders, granules, coated particles or coated tablets, tablet, pills, troches, gastro-resistant tablets and capsules and dispersible strips and films.

In another particular embodiment, the liquid formulation is selected from the group consisting of oral solutions, suspensions, emulsions and syrups.

Likewise, various systems are known that can be used for sustained-release administration of any of the probiotic, pharmaceutical or nutritional compositions of the invention, including, for example, the encapsulation in liposomes, microbubbles, microparticles or microcapsules and the like. The suitable sustained-release forms as well as materials and methods for their preparation are well known in the state of the art. Thus, the orally administrable form of any of the probiotic compositions of the invention is in a sustained-release form further comprising at least one coating or matrix. The sustained release coating or matrix includes, without limitation, natural semisynthetic or synthetic polymers, water-insoluble or modified, waxes, fats, fatty alcohols, fatty acids, natural, semisynthetic or synthetic plasticizers or a combination of two or more of the same. Enteric coatings can be applied using conventional processes known to those skilled in the art.

In addition to what has been described above, the present invention also encompasses the possibility that any of the probiotic compositions, probiotic pharmaceutical or nutritional compositions of the present invention (as reflected in any aspect or embodiment described throughout the present specification) may be administered to a subject together with other components or compounds, although these are not part of the probiotic compositions, probiotic pharmaceutical or nutritional compositions of the present invention. In this sense, in the event that the composition of the invention is formulated as a nutritional composition, said nutritional composition may be a food or be incorporated into a food or food product intended for both human and animal consumption. Thus, in a particular embodiment, the nutritional composition is selected from between a food (which may be a food for specific nutritional purposes or medicinal food) and a nutritional supplement.

In the present invention, the term "nutritional composition" refers to that food, which regardless of providing nutrients to the subject who consumes it, beneficially affects one or more functions of the body, so as to provide better health and wellness. In the present invention, said nutritional composition is intended to ease, reduce, treat and/or prevent Rosacea.

The term "supplement", synonymous with any of the terms "dietary supplement", "nutritional supplement", "food supplement", or "alimentary supplement" or "alimentary complement" refers to products or preparations whose purpose is to supplement the normal diet consisting of sources of concentrated nutrients or other substances with a nutritional or physiological effect. In the present invention, the "substance" which has a nutritional or physiological effect on the individual when the alimentary complement is ingested are *L. rhamnosus* CECT 30579 and/or *B. longum* CECT 30615, which are part of any of the compositions of the present invention. The food supplement may be in single or combined form and be marketed in dosage form, i.e. in capsules, pills, tablets and other similar forms, sachets of powder, ampoules of liquids and drop dispensing bottles and other similar forms of liquids and powders designed to be taken in a single amount.

There is a wide range of nutrients and other elements that may be present in alimentary complements including, among others, vitamins, minerals, amino acids, essential fatty acids, fibre, enzymes, plants and plant extracts. Since their role is to complement the supply of nutrients in a diet, they should not be used as a substitute for a balanced diet and intake should not exceed the daily dose expressly recommended by the doctor or nutritionist. The probiotic composition can also be part of the so-called "food for special groups", i.e. foods that meet specific nutritional needs.

Examples of foods that may comprise the compositions or probiotic compositions of the invention (microorganisms *L. rhamnosus* CECT 30579 and/or *B. longum* CECT 30615 (or strains derived therefrom)), include, but are not limited to, feed, dairy products, vegetable products, meat products, snacks, chocolates, drinks, baby food, cereals, fried foods, industrial bakery products and biscuits. Examples of milk products include, but are not limited to, products derived from fermented milk (for example, but not limited to, yogurt or cheese) or non-fermented milk (for example, but not limited to, ice cream, butter, margarine or whey). The vegetable product is, for example, but not limited to, a cereal in any form of presentation, fermented (for example, soy yogurt, oat yogurt, etc.) or unfermented, and a snack. The beverage may be, but is not limited to, non-fermented milk. In a particular embodiment, the food product or food is selected from the group consisting of fruit or vegetable juices, ice cream, infant formula, milk, yogurt, cheese, fermented milk, powdered milk, lyophilized or air-dried products (suitable for reconstitution with a liquid vehicle), cereals, baked goods, milk-based products, meat products and beverages.

As understood by those skilled in the art, any of the compositions or probiotic compositions of the invention may be formulated as forming part of personal care products or cosmetic products to be administered to the subject e.g. topically. Therefore, in a particular embodiment, any of the compositions or probiotic compositions of the invention can be a personal care product or a cosmetic product.

Non-limiting examples of personal care products include bar soap, liquid soap (e.g., hand soap), hand sanitizer (including rinse off and leave-on alcohol based and aqueous-based hand disinfectants), cotton swabs and pads, shaving cream, talcum powder, toiled paper, preoperative skin disinfectant, wet paper, cleansing wipes, disinfecting wipes, body wash, acne treatment products, antifungal diaper rash cream, antifungal skin cream, shampoo, conditioner, cosmetics deodorant, antimicrobial creams, body lotion, hand cream, topical cream, aftershave lotion, skin toner, oral hygiene products, and sunscreen lotion. The compositions of the invention may also be applied to wound care items, such as, but not limited to, wound healing ointments, creams, and lotions, wound coverings, burn wound cream, bandages, tape, and steri-strips, and medical articles such as medical gowns, caps, face masks, and shoe-covers, surgical drops, etc. In one particular embodiment, the personal care product is a topical cream or gel.

Additionally, any of the compositions, probiotic compositions, pharmaceutical or nutritional compositions of the present invention (as reflected in any aspect or embodiment described throughout the present specification), may comprise other microorganisms in addition to *L. rhamnosus* CECT 30579 and/or *B. longum* CECT 30615. Thus, in a particular embodiment, any of the compositions, probiotic, pharmaceutical or nutritional compositions of the present invention (as reflected in any aspect or embodiment described throughout the present specification) may further comprise one or more microorganism selected, among others, from the following group: *Lacticaseibacillus sp., Streptococcus sp., Bifidobacterium sp., Saccharomyces sp.,* and combinations thereof.

In another particular embodiment, any of the compositions of the invention are administered to a subject through the diet.

In another particular embodiment, any of the compositions of the invention are administered to a subject via oral administration.

As understood by one skilled in the art, the microorganisms *L. rhamnosus* CECT 30579 and/or *B. longum* CECT 30615 (or any microorganisms derived therefrom) have to be present in the probiotic, pharmaceutical or nutritional compositions of the present invention (as reflected in any aspect or embodiment described throughout the present specification) in an effective amount, particularly in a therapeutically effective amount, so that they can exert their effect of easing, reducing, treating and/or preventing Rosacea. In the present invention "effective amount" or "therapeutically effective amount" is that amount of the component or compound of the the probiotic, pharmaceutical or nutritional compositions of the present invention, which when administered to a subject, is sufficient to produce the desired effect. Said component or compound of the probiotic, pharmaceutical or nutritional compositions of the present invention (as reflected in any aspect or embodiment described throughout the present specification), refers to the microorganisms *L. rhamnosus* CECT 30579 and/or *B. longum* CECT 30615. The therapeutically effective amount will vary depending on, for example, age, body weight, general health, sex and diet of the subject, as well as according to the mode and time of administration, excretion rate or drug combination, among other factors.

In another particular embodiment, the total concentration of microorganisms *L*. *rhamnosus* CECT 30579 and/or *B. longum* CECT 30615 (or any microorganisms derived therefrom), in any of the compositions of the invention is between 10³ and 10¹² cfu, preferably between 10⁶ and 10¹¹ cfu, between 10⁶ and 10¹⁰ cfu, between 10⁷ and 10¹¹ cfu, between 10⁷ and 10¹⁰ cfu, between 10⁸ and 10¹¹ cfu, between 10⁸ and 10¹⁰ cfu, particularly approximately 10⁹ or 10¹⁰ cfu.

In another particular embodiment, the dose of administration of microorganisms *L*. *rhamnosus* CECT 30579 and/or *B. longum* CECT 30615 (or any microorganisms derived therefrom), in the composition is between 10⁶ and 10¹² cfu/day, between 10⁶ and 10¹⁰ cfu/day, between 10⁷ and 10¹¹ cfu/day, between 10⁷ and 10¹⁰ cfu/day, between 10⁸ and 10¹¹ cfu/day, between 10⁸ and 10¹⁰cfu/day, particularly approximately 10⁹ or 10¹⁰cfu/day, and in another even more particular embodiment, the administration regimen is at least once daily, in particular twice daily, and more in particular, three times a day, one with each food intake (breakfast, lunch and dinner).

In another particular embodiment of any of the probiotic pharmaceutical or nutritional compositions of the present invention (as reflected in any aspect or embodiment described throughout the present specification), the concentration of *L. rhamnosus* CECT 30579 is at least thirty percent (30%) with respect to the total concentration of microorganisms present in the composition, particularly at least 31 %, 32%, 33%, 34%,35%, 36%, 37%, 38%, 39%, 40%, 41%, 42%, 43%, 44%, 45%, 46%, 47%, 48%, 49%, 50% relative to the total concentration of microorganisms present in any of the compositions of the invention. In another embodiment, the concentration of *L*. *rhamnosus* CECT 30579 is at least 50%, 55%, 60%, 65%, 70%, 75%, 80%, 85%, 90%, 91%, 92%, 93%, 94%, 95%, 96%, 97%, 98%, or 99%, preferably at least 60%, 65%, 70%, 75% or 80%, most preferred at least 85%, 90%, 91%, 92%, 93%, 94%, 95%, 96% with respect to the total amount of microorganisms present in any of the compositions of the invention.

In another embodiment of present invention, any of the probiotic pharmaceutical or nutritional compositions comprise between 10³ to 10¹² colony forming units (cfu), more preferred at least about 10⁶ cfu, most preferred at least about 10⁹ cfu of *L. rhamnosus* CECT 30579.

In one embodiment of present invention, the concentration of *L. rhamnosus* CECT 30579 is of at least 90% or at least 95% or of 100% with respect to the total amount of microorganisms present in the compositions of present invention. Preferably, this concentration of *L. rhamnosus* CECT 30579 provides for an amount of between 10³ to 10¹² colony forming units (cfu), more preferred at least about 10⁶ cfu, most preferred about 10⁹ cfu of *L. rhamnosus* CECT 30579 in said compositions.

In another particular embodiment of any of the probiotic pharmaceutical or nutritional compositions of the present invention (as reflected in any aspect or embodiment described throughout the present specification), the concentration of *B. longum* CECT 30615 is at least thirty percent (30%) with respect to the total concentration of microorganisms present in the composition, particularly at least 31%, 32%, 33%, 34%, 35%, 36%, 37%, 38%, 39%, 40%, 41%, 42%, 43%, 44%, 45%, 46%, 47%, 48%, 49%, 50% relative to the total concentration of microorganisms present in any of the compositions of the invention. In another embodiment, the concentration of *B. longum* CECT 30615 is at least 50%, 55%, 60%, 65%, 70%, 75%, 80%, 85%, 90%, 91%, 92%, 93%, 94%, 95%, 96%, 97%, 98%, or 99%, preferably at least 60%, 65%, 70%, 75% or 80%, most preferred at least 85%, 90%, 91%, 92%, 93%, 94%, 95%, 96% with respect to the total amount of microorganisms present in any of the compositions of the invention.

In another embodiment of present invention, any of the probiotic pharmaceutical or nutritional compositions comprise between 10³ to 10¹² colony forming units (cfu), more preferred at least about 10⁶ cfu, most preferred at least about 10⁹ cfu of *B. longum* CECT 30615.

In one embodiment of present invention, the concentration of *B. longum* CECT 30615 is of at least 90% or at least 95% or of 100% with respect to the total amount of microorganisms present in the compositions of present invention. Preferably, this concentration of B. *longum* CECT 30615 provides for an amount of between 10³ to 10¹² colony forming units (cfu), more preferred at least about 10⁶ cfu, most preferred about 10⁹ cfu of *B. longum* CECT 30615 in said compositions.

In a preferred embodiment of present invention, the concentration of *B. longum* CECT 30615 is between 40% to 60% with respect to the total amount of microorganisms present in the compositions of present invention and the concentration of *L. rhamnosus* CECT 30579 is between 40% to 60% with respect to the total amount of microorganisms present in the compositions of present invention so that the combination of both amounts to 100% of the total amount of microorganisms present in the compositions of present invention. Preferably, this concentration of *B. longum* CECT 30615 and *L. rhamnosus* CECT 30579 provides for an amount of between 10³ to 10¹² colony forming units (cfu), more preferred at least about 10⁶ cfu, most preferred about 10⁹ cfu of each of the strains *B. longum* CECT 30615 and *L. rhamnosus* CECT 30579 in said compositions.

In the most preferred embodiment the concentration of *B*. *longum* CECT 30615 is of about 50% with respect to the total amount of microorganisms present in the compositions of present invention and the concentration of *L. rhamnosus* CECT 30579 is of about 50% with respect to the total amount of microorganisms present in the compositions of present invention so that the combination of both amounts to 100% of the total amount of microorganisms present in the composition of present invention. In one embodiment this concentration of *B. longum* CECT 30615 and *L. rhamnosus* CECT 30579 provides for an amount of between 10³ to 10¹² colony forming units (cfu), more preferred at least about 10⁶ cfu, most preferred about 10⁹ cfu of *B. longum* CECT 30615 *L. rhamnosus* CECT 30579 of each of the strains *B*. *longum* CECT 30615 and *L*. *rhamnosus* CECT 30579 in said compositions. In another embodiment this concentration of *B. longum* CECT 30615 and *L. rhamnosus* CECT 30579 provides for an amount of between 10³ to 10¹² colony forming units (cfu), more preferred at least about 10⁶ cfu, most preferred about 10⁹ cfu of *B. longum* CECT 30615 *L. rhamnosus* CECT 30579 of B. *longum* CECT 30615 and *L. rhamnosus* CECT 30579 together in said compositions.

In one embodiment *B*. *longum* CECT 30615 and *L. rhamnosus* CECT 30579 are the only bacteria present in the composition.

In a further embodiment the composition comprises further bacterial strains in the same amounts as specified above for *B. longum* CECT 30615 and *L. rhamnosus* CECT 30579. Said additional bacterial strains can be other *B. longum* and/or *L. rhamnosus* strains.

In yet a further embodiment of the present invention the probiotic pharmaceutical or nutritional compositions of the present invention further comprise at least one bulking agent and/or at least one anti-caking agent and/or at least one coating agent and optionally at least one opacifier.

Preferably said at least one bulking agent of present invention is maltodextrin and/or said at least one coating agent of present invention is gelatin and/or said at least one opacifier is titanium dioxide.

The at least one anti-caking agent of present invention can be selected from the group consisting of magnesium stearate, calcium stearate, silica, silicates, such as sodium or calcium silicate, talc, flour, starch, or combinations thereof.

In a preferred embodiment the bulking agent is maltodextrin, the anti-caking agent is magnesium stearate, the coating agent is gelatin and the opacifier is titanium dioxide.

Specifically, any of the probiotic compositions, probiotic pharmaceutical or nutritional compositions of the present invention (as reflected in any aspect or embodiment described throughout the present specification), are used in a method of treating or preventing Rosacea in a subject or individual, comprising, particularly orally, administering to said subject or individual, an effective amount of the composition thereby treating or preventing said Rosacea. As already indicated previously, it is noted that orally suitable liquid preparations to be used for the compositions of the present invention, may be in the form of, for example, aqueous or oily suspensions, solutions, emulsions, syrups or elixirs, or may be presented as a dry product, such as a lyophilized (freeze-dried) or air-dried product, for constitution with water or other suitable liquid vehicles before use. Such liquid preparations may contain conventional additives such as suspending agents, emulsifying agents, non-aqueous vehicles (which may include edible oils), or preservatives.

In one further embodiment, the method of the invention for treating Rosacea in a subject, may further comprise the step of sequentially, subsequently or simultaneously administering to said subject an effective amount of an additional agent, such as isotrenitoin, salicylic acid, witch hazel or Benzoyl peroxide, topical or oral retinoid, spironolactone, an oral contraceptive, azaleic acid, glycolic acid, topical or oral antibiotics, sulfa-based anti-biotics, spf/sunblock, moisturizers or a combination thereof in other embodiments. A person skilled in the art will readily recognize that the components of any treatment composition may be adjusted and optimized based on the symptoms exhibited by the subject, their severity and potential synergistic or antagonistic interactions among the components of such treatment, without exceeding the scope of the invention.

In one further embodiment, the methods and compositions of the invention are used as a supplementary or adjuvant treatment for Rosacea, wherein an effective amount of any of the compositions of the present invention, as defined herein, are typically administered orally, either singly or in combination (simultaneously, sequentially or subsequently) with another compound or compounds such isotrenitoin, salicylic acid, witch hazel or Benzoyl peroxide, topical or oral retinoid, spironolactone, an oral contraceptive, azaleic acid, glycolic acid, topical or oral antibiotics, sulfa-based antibiotics, spf/sunblock, or moisturizers. In particular, with antibiotics such as but not limited to topical or oral antibiotics, sulfa-based antibiotics, ivermectin or isotretinoin. The administration can be carried out in one embodiment, in single unit dosage form with continuous therapy or in another embodiment, in single dose therapy ad libitum. Other embodiments of administration are effective for treating the Rosacea conditions. In other embodiments, any of the compositions of the invention are used when relief of symptoms is specifically required, or, in one embodiment, imminent. Lastly, any of the compositions of the invention may be further used in another embodiment, as a continuous or prophylactic treatment.

Throughout the description and claims the word "comprise" and its variants are not intended to exclude other technical characteristics, additives, components or steps. For those skilled in the art, other objects, advantages and characteristics of the invention will become apparent in part from the description and partly from the practice of the invention. The following examples and figures are provided by way of illustration and are not intended to limit the scope of the present invention.

### EXAMPLES

### EXAMPLE 1: STRAIN SELECTION

The species *Lacticaseibacillus rhamnosus* (formerly *Lactobacillus rhamnosus*), is one of the most widely used bacteria for its probiotic properties, both digestive and anti-inflammatory. In addition, *L. rhamnosus* is included in the list of bacterial species with QPS (Qualified Presumption of Safety) status by the European Food Safety Agency. For the selection of the *L. rhamnosus* CECT 30579 strain, its anti-inflammatory properties were compared with others of the same species.

The immune cell cultures treated with the probiotic strains CET 30579 showed a significant decrease in TNF-alpha (a pro-inflammatory cytokine) higher increase of IL-10 and a higher reduction of LPS and bacterial DNA than other *L. rhamnosus* strains. On the other hand, exposure to *L. rhamnosus* CECT 30579 to PBMC cultures did not increase IL-6 production, in contrast to the response generated by other *L. rhamnosus* strains tested in this study. In terms of anti-inflammatory cytokine production, all strains, including *L. rhamnosus* CECT 30579) significantly increased TGF-β production, although the increase was less pronounced in the case of the *L. rhamnosus* strain CECT 30579 (unpublished data). In addition, the same strains were tested in a small study including 5 patients per probiotic strain, also demonstrating that *L. rhamnosus* strain CECT 30579 was the one with the best clinical results according to the subjective sensation of the patients.

In the case of *Bifidobacterium longum* some strains were previously tested in an in vitro study with two other different strains of this species and the one selected was *Bifidobacterium longum* CECT 30615 which showed greater systemic anti-inflammatory properties with a greater increase in IL-10 levels than the other B. *longum* strain.

Following these results both strains were tested in a small pilot study involving a total of 20 subjects who took in blocks of 5 patients either placebo, the probiotic *L. rhamnosus* CECT 30579, the probiotic *B*. *longum* CECT 30615 or the mixture of both probiotics. In the group receiving only probiotic *L. rhamnosus* CECT 30579 one patient of five (20%) improved in at least one category in the IgA score. In the group receiving only probiotic *B. longum* CECT 30615 two patients of five (40%) improved in at least one category in the IgA score. However, when the mixture of the two probiotics was used 4 of the 5 patients (80%) improved in some of the scales.

These data suggested a potentiating effect of the two probiotics used together, hence it was the two strains together that were selected for a double-blind confirmatory pilot study using the mixture compared with data from a placebo intervention group in cases with Rosacea. The data from this study with analysis of the results of the first 50 cases included in the study are shown in the following section.

### EXAMPLE 2: IMMUNOMODULATORY CAPACITY OF L. RHAMNOSUS CECT 30579 AND B. LONGUM CECT 30615

### Cultures with immunocompetent cells

Peripheral blood mononuclear cells (PBMC) were used to evaluate the immunomodulatory capacity of the selected strain of *L. rhamnosus* CECT 30579. PBMC cells (StremCell, Vancouver, Canada) had been isolated from volunteers participating in the Rosacea clinical trial and after thawing, were resuspended in RPMI 1640 medium supplemented with 10% (v/v) fetal bovine serum, 1% (v/v) HEPES and 1% (v/v) antibiotic cocktail. For the assay, cells were seeded in 24-well culture plates at a density of 1 × 106 cells per well. The cells were incubated at 37°C for 24 hours. After this time, PBMC were stimulated for 16 hours with a suspension including *L. rhamnosus* strain CECT 30579 at a 1:1 ratio (cells:bacteria), thus adding 1 × 106 CFU of *L. rhamnosus* per well. Lipopolysaccharide (LPS) from Salmonella typhimurium (1 ug/ml) was used as a positive control. A phosphate buffered saline (PBS) dilution was used as a negative control. After 16h of stimulation, supernatants were collected and kept frozen at -80°C until used for cytokine quantification.

The MILLIPLEX^{®}Human TH17 Bead Panel kit (HTH17MAG, Merck) was used, which allowed simultaneous quantification of the cytokines IFN-γ, IL-6, TNF-α and TGF-β. For this analysis, a Luminex MAGPIX^{®} System equipped with xPONENT^{®} analysis software was used. The assay was performed with 4-6 replicates per condition.

### Statistical analysis

Student's t-tests were performed comparing the positive control (Isobutylmethylxanthine or LPS treatment) or *L. rhamnosus* CECT 30579 with the negative control (PBS). All statistical analyses were performed using GraphPad Prism 9.5.1. Results are reported as mean ± standard error with each point being a biologic replicate. Values of p < 0.05 were considered statistically significant.

### RESULTS

### Evaluation of immunomodulatory capacity of L. rhamnosus CECT 30579

The results of exposure of *L. rhamnosus* CECT 30579 to PBMC cultures are shown in Figure 1. The immune cell cultures treated with the probiotic bacteria showed a significant decrease in TNF-alpha (a pro-inflammatory cytokine) (Fig.1A). On the other hand, exposure to *L. rhamnosus* CECT 30579 to PBMC cultures did not increase IL-6 production, in contrast to the response generated by LPS (Fig.1B). In terms of anti-inflammatory cytokine production, both treatments (LPS and *L. rhamnosus* CECT 30579) significantly increased TGF-β production, although the increase was less pronounced in the case of the bacteria (Fig.1C).

### Evaluation of the Immunomodulatory Capacity of B. longum CECT 30615 in PBMCs

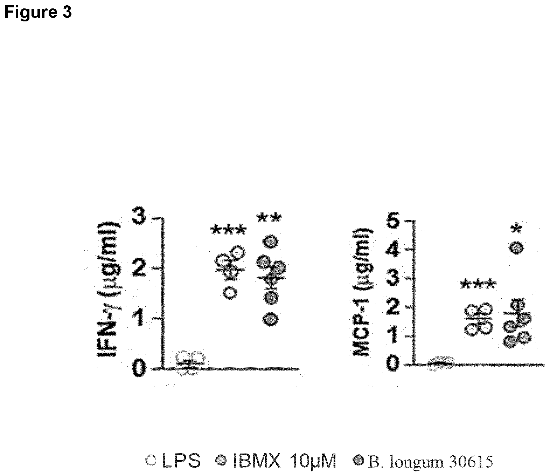

The results of *B. longum* CECT 30615 exposure to PBMC cultures are shown in Figure 2. The immune cell cultures treated with the bacterium, as well as the cultures exposed to LPS, showed a significant increase in IFN-γ (a pro-inflammatory cytokine involved in macrophage activation) and MCP-1 (involved in monocyte migration) although in this second case the effect was lower with the probiotic than with LPS. Regarding anti-inflammatory cytokine production, both treatments (LPS and *B*. *longum* CECT 30615) significantly increased TGF-β production (Fig. 3).

### DISCUSSION

The ability to modulate the immune system response has been the subject of numerous studies and has been proposed as one of the mechanisms explaining the probiotic properties of certain bacteria (Thoda et al., 2023). The results of our study are in line with previous work showing that some strains enhance the anti-inflammatory response and also reduce the levels of pro-inflammatory cytokines (TNF-α expression) in PBMC cultures pre-stimulated with LPS (Barigela et al., 2021). In our study we have also observed the anti-inflammatory potential of *L. rhamnosus* CECT 30579 in view of the lower induction of IL-6 production than that elicited by LPS, together with the increase of the anti-inflammatory cytokine TGF-β. When evaluating the effect of *B*. *longum* 30615 on other cytokines, we demonstrated an effect on the production of pro-inflammatory cytokines such as interferon gamma (IFN-γ) with both LPS and probiotic, although there is a lower pro-inflammatory induction of MCP-1 (involved in monocyte migration) with probiotic than with LPS. Finally, both LPS and *B. longum CECT 30615* have an induction in the production of anti-inflammatory cytokines with a significant increase in TGF-β production.

These different results can be attributed to the existence of differences between the two probiotic strains tested, and their possible action through different mechanisms.

### EXAMPLE 3: A RANDOMIZED, DOUBLE-BLIND, PLACEBO-CONTROLLED CLINICAL TRIAL TO EVALUATE THE EFFECT OF A PROBIOTIC MIXTURE ON THE EVOLUTION AND TREATMENT OF ROSACEA

### Brief Summary

The clinical trial has a randomized, double-blind, placebo-controlled design, in which the aim is to evaluate the effect of a probiotic mix, with a 12-week treatment, on the evolution of Rosacea.

### Detailed Description

These 12 weeks of treatment are structured into three visits: Visit 1 (initial; week 0), Visit 2 (intermediate; week 6) and Visit 3 (final; week 12). The three visits that make up the study and the actions to be carried out in each of them are described below:

### Visit 1 (initial; week 0)

Once the informed consent is signed, a doctor trained for the study performs the initial interview where it is verified that the patient meets all the inclusion criteria and none of the exclusion criteria and their medical history. The investigator will proceed to assign the patient the next study participant number and, according to a previously prepared randomization list, the treatment that they will receive during the study will be assigned. The investigator will assess the severity and symptoms of Rosacea according to the Investigator's Global Assessment (IGA) and Clinician Erythema Assessment (CEA) scales. The patient will also complete a questionnaire to assess the impact on quality of life due to the disease: Dermatology Life Quality Index (DLQI).

### Visit 2 (intermediate; week 6)

In this intermediate visit, in addition to assessing the symptoms of the disease with the detailed scales, the investigator will record the adverse events reported by the patient, as well as the concomitant treatments used. Also, the treatment compliance rate will be calculated by counting the capsules returned by the patient.

### Visit 3 (final; week 12)

In the final visit, the same actions will be carried out as in the intermediate visit, evaluating the severity of the disease and the remaining capsules will be collected, to calculate treatment compliance, concomitant treatments and adverse events.

### Visit 4 (post-treatment follow-up; week 24)

During this visit, the symptoms of the disease will be assessed with the two scales used in the study and the treatments that the patient has required during the 12 weeks after the end of the study treatment will be counted.

### Interventional product

- Dietary Supplement: Probiotic mixture
   ∘ Oral capsule consumption once a day for 12 weeks
- Dietary Supplement: Placebo
   ∘ Oral capsule consumption once a day for 12 weeks

### Study Arms

- Experimental: Probiotic group (30 patients)
   ∘ Probiotic mixture in oral capsule format with *B*. *longum CECT 30615* and *L. rhamnosus* CECT 30579 in concentrations equal to or greater than 1×10⁹ cfu/dose for each of the strains, wherein the dose contains the strains at equal amounts, i.e. 50% *B*. *longum CECT 30615* and 50% *L. rhamnosus* CECT 30579.
   ∘ Interventions:
      ▪ Dietary Supplement: Probiotic mixture

- Placebo Comparator: Placebo group (30 patients)
   ∘ Maltodextrin in oral capsule format
   ∘ Interventions:
      ▪ Dietary Supplement: Placebo

### Eligibility Criteria

### Inclusion Criteria:

- Patients of both sexes of an age equal to or greater than 18 years.
- Patients diagnosed with papulopustular Rosacea with a minimum score of 2 points according to the Investigator's Global Assessment (IGA) index.
- Signature of informed consent by the patient

### Exclusion Criteria:

- Pregnant, lactating, and/or women who do not agree to use an effective contraceptive method during the development of the study.
- Allergy and/or intolerance to any of the components of the product under study.
- Consumption of antibiotics in the previous two weeks.
- Consumption of probiotics in the previous two months.
- Isotretinoin use in the previous six months.
- Light procedures (IPL, laser, Kleresca) in the previous three months.
- Participation in other clinical studies in the previous two months.
- Other dermatological pathologies.
-

### Sex/Gender

### Sexes Eligible for the Study: 18 Years and older

### Outcome Measures

1. Number and percentage of cases responding to the treatment according to the Investigator's Global Assessment (IGA) index score, comparing the baseline with the visits at 6 and 12 weeks
   IGA index measures the severity of Rosacea
   Score range is from 0 to 6 according to the severity of the disease
   0=clear; 1=minimum; 2=mild; 3=mild-moderate; 4=moderate; 5=moderate-severe; 6=severe
   It will be considered a treatment response in IGA scale when the participant improves by at least 2 categories in this index and the final score is 0-1 points (clear and almost clear)
   [Time Frame: 12 weeks]
2. Number and percentage of cases responding to the treatment according to the IGA index score, analyzing the cases that received any topical or systemic treatment, comparing the baseline with the visits at 6 and 12 weeks
   The treatments considered will be any topical or systemic treatment
   [Time Frame: 12 weeks]
3. Number and percentage of cases responding to the treatment according to the IGA index score, analyzing the cases that received systemic treatment, comparing the baseline with the visits at 6 and 12 weeks
   The treatments considered will be any systemic treatment
   [Time Frame: 12 weeks]
4. Number and percentage of cases that need any treatment (topical or systemic) and number of days during the treatment period (12 weeks)
   The number of treatment days and the number of participants prescribed treatment by the dermatologist will be counted
   The treatments considered will be any topical or systemic treatment
   [Time Frame: 12 weeks]
5. Number and percentage of cases that need systemic treatment and number of days during the treatment period (12 weeks)
   The number of treatment days and the number of participants prescribed systemic treatment by the dermatologist will be counted
   The treatments considered will be any systemic treatment
   [Time Frame: 12 weeks]
6. Number and percentage of cases that need topical treatment and number of days during the treatment period (12 weeks)
   The number of days and the number of participants prescribed topical treatment by the dermatologist will be counted
   The treatments considered will be any topical treatment
   [Time Frame: 12 weeks]
7. Number and percentage of cases responding to the treatment according to the Dermatology Life Quality Index (DLQI) at 6 and 12 weeks
   DLQI measures the impact Rosacea has on your quality of life
   The index has 10 questions. Each question answered with "Very much" is counted with 3 points, "A lot" with 2, "Little" with 1, and "Not at all" as 0.
   The score obtained can range between 0 and 30 points, this being the worst possible score.
   It will be considered a treatment response in DLQI scale when the score is 0-5 points
   (no effect or mild effect in the quality of life)
   [Time Frame: 12 weeks]
8. Number and percentage of cases responding to the treatment according to the IGA index score, comparing the baseline with the visit at 24 weeks (post-treatment follow-up)
   IGA index measures the severity of Rosacea
   Score range is from 0 to 6 according to the severity of the disease
   0=clear; 1=minimum; 2=mild; 3=mild-moderate; 4=moderate; 5=moderate-severe; 6=severe
   It will be considered a treatment response when the participant improves by at least 2 categories in this index and the final score is 0-1 points (clear and almost clear)
   [Time Frame: 24 weeks]
9. Number and percentage of cases requiring any treatment (topical or systemic), along with the number of days, covering the total period (24 weeks) and the post-treatment follow-up period (from week 12 to 24)
   The number of treatment days and the number of participants prescribed treatment by the dermatologist will be counted
   The treatments considered will be any topical or systemic treatment
   [Time Frame: 24 weeks]
10. Number and percentage of cases requiring systemic treatment, along with the number of days, covering the total period (24 weeks) and the post-treatment follow-up period (from week 12 to 24)
   The number of treatment days and the number of participants prescribed systemic treatment by the dermatologist will be counted
   The treatments considered will be any systemic treatment
   [Time Frame: 24 weeks]
11. Number and percentage of cases requiring topical treatment, along with the number of days, covering the total period (24 weeks) and the post-treatment follow-up period (from week 12 to 24)
   The number of treatment days and the number of participants prescribed topical treatment by the dermatologist will be counted
   The treatments considered will be any topical treatment
   [Time Frame: 24 weeks]
12. Study treatment compliance rate at 6 and 12 weeks.
   The treatment adherence is collected, checking the number of remaining capsules in each of the visits
   [Time Frame: 12 weeks]
13. Number of adverse events at 6 and 12 weeks
   Adverse events reported by patients during follow-up at week 6 and week 12 visits are recorded
   [Time Frame: 12 weeks]

### RESULTS

An intermediate analysis of the most relevant outcomes is conducted, including 50 patients and comparing data between the initial visit (visit 1-baseline) and the end-of-treatment visit (visit 3 -12 weeks visit)
1. Number and percentage of cases responding to the treatment according to the Investigator's Global Assessment (IGA) index score, comparing the baseline with the 12 weeks visit
   In the probiotic group, the number and percentage of cases responding to the treatment according to the IGA score were 17/25 (68%), while in the placebo group, it was 12/25 (48%). This 20% improvement in the cases taking the probiotic mixture was clinically significant.
   1.1 Number and percentage of cases responding to the treatment according to the IGA index score, analyzing the cases that received any topical or systemic treatment, comparing the baseline with the 12 weeks visit.
      Of the 50 patients analyzed, 33 received some form of topical or systemic treatment. In the probiotic group, the number and percentage of cases responding to the treatment according to the IGA score (analyzing the 33 cases that received any topical or systemic treatment) were 13/16 (81%), while in the placebo group, it was 9/17 (53%). This 28% improvement in the cases taking the probiotic mixture was clinically significant.
   1.2 Number and percentage of cases responding to the treatment according to the IGA index score, analyzing the cases that received systemic treatment, comparing the baseline with the 12 weeks visit.
      Of the 50 patients analyzed, 25 received some form of systemic treatment. In the probiotic group, the number and percentage of cases responding to the treatment according to the IGA score (analyzing the 25 cases that received any systemic treatment) were 10/12 (83%), while in the placebo group, it was 8/13 (62%). This 21% improvement in the cases taking the probiotic mixture was clinically significant.
2. Number and percentage of cases that need any treatment (topical or systemic) and number of days during the treatment period (12 weeks)
   The total follow-up days in each group were 2100 (84 days x 25 patients).
   The days of treatment (topical or systemic) were 1071 in the probiotic group and 1092 in the placebo group, representing a reduction of 21 days in total treatment in the patients of the probiotic group.
   2.1. Number and percentage of cases that need topical treatment and number of days during the treatment period (12 weeks)
   The days of topical treatment were 987 in the probiotic group and 1071 in the placebo group, representing a reduction of 84 days in topical treatment in the patients of the probiotic group. This reduction in the cases taking the probiotic mixture was clinically significant.

### REFERENCES

Barigela A, Bhukya B. Probiotic Pediococcus acidilactici strain from tomato pickle displays anticancer activity and alleviates gut inflammation in-vitro. 3 Biotech. 2021;11(1):23. doi: 10.1007/s13205-020-02570-1.
Barakji YA, Rønnstad ATM, Christensen MO, Zachariae C, Wienholtz NKF, Halling AS, Maul JT, Thomsen SF, Egeberg A, Thyssen JP. Assessment of Frequency of Rosacea Subtypes in Patients With Rosacea: A Systematic Review and Meta-analysis. JAMA Dermatol. 2022 Jun 1;158(6):617-625. doi: 10.1001/jamadermatol.2022.0526. PMID: 35385049; PMCID: PMC8988027.
Benyacoub J., Bosco N., Blanchard C., Demont A., Phillippe D., Castiel-Higounenc I. Immune modulation property of Lacticaseibacillus paracasei NCC2461 (ST11) strain and impact on skin defenses. Benef Microbes. 2014; 5:129-136.
Bowe W.P., Logan A.C. Acne vulgaris, probiotics and the gut-brain-skin axis: back to the future? Gut Pathog. 2011;3:1.
Clanner-Engelshofen, Benjamin & Bernhard, Dominik & Dargatz, Sonja & Flaig, Michael & Gieler, Uwe & Kinberger, Maria & Klövekorn, Winfried & Kuna, Anne-Charlotte & Läuchli, Severin & Lehmann, Percy & Nast, Alexander & Pleyer, Uwe & Schaller, Martin & Steinhoff, Martin & Schwennesen, Thomas & Werner, Ricardo & Zierhut, Manfred & Reinholz, Markus. (2022). S2k guideline: Rosacea. JDDG: Journal der Deutschen Dermatologischen Gesellschaft. 20. 10.1111/ddg.14849.
Dirschka T, Micali G, Papadopoulos L, Tan J, Layton A, Moore S. Perceptions on the Psychological Impact of Facial Erythema Associated with Rosacea: Results of International Survey. Dermatol Ther (Heidelb). 2015 Jun;5(2):117-27. doi: 10.1007/s13555-015-0077-2. Epub 2015 May 29. PMID: 26022994; PMCID: PMC4470961.
Eguren C, Navarro-Blasco A, Corral-Forteza M, Reolid-Pérez A, Setó-Torrent N, García-Navarro A, Prieto-Merino D, Núñez-Delegido E, Sánchez-Pellicer P, Navarro-López V. A Randomized Clinical Trial to Evaluate the Efficacy of an Oral Probiotic in Acne Vulgaris. Acta Derm Venereol. 2024 May 15;104:adv33206. doi: 10.2340/actadv.v104.33206. PMID: 38751177; PMCID: PMC11110809.
Gallo RL, Granstein RD, Kang S, Mannis M, Steinhoff M, Tan J, Thiboutot D. Standard classification and pathophysiology of rosacea: The 2017 update by the National Rosacea Society Expert Committee. J Am Acad Dermatol. 2018 Jan;78(1):148-155. doi: 10.1016/j.jaad.2017.08.037. Epub 2017 Oct 28. PMID: 29089180.
Gether L, Overgaard LK, Egeberg A, Thyssen JP. Incidence and prevalence of rosacea: a systematic review and meta-analysis. Br J Dermatol. 2018 Aug;179(2):282-289. doi: 10.1111/bjd.16481. Epub 2018 May 31. PMID: 29478264.
Gueniche A., Phillippe D., Bastien P., Reuteler G., Blum S., Castiel-Higounenc I. Randomised double-blind placebo-controlled study of the effect of Lacticaseibacillus paracasei NCC 2461 on skin reactivity (Benef Microbes. 2014;5:137-145.
Hacini-Rachinel G., Gheit H., Le Luduec J.B., Dif F., Nancey S., Kaiserlian D. Oral probiotics, including different species of Lacticaseibacillus, control skin inflammation by acting on both effector and regulatory T cells. PLoS One. 2009;4:e4903.
Navarro-López V, Núñez-Delegido E, Ruzafa-Costas B, Sánchez-Pellicer P, Agüera-Santos J, Navarro-Moratalla L. Probiotics in the Therapeutic Arsenal of Dermatologists. Microorganisms. 2021 Jul 15;9(7):1513. doi: 10.3390/microorganisms9071513. PMID: 34361948; PMCID: PMC8303240.
Peyton Morss-Walton, Jean S. McGee,Rosacea, not just skin deep: Understanding thesystemic disease burden,Clinics in Dermatology,Volume 39, Issue 4,2021,Pages 695-700,ISSN 0738-081X,https://doi.org/10.1016/j.clindermatol.2020.08.006.
Salleras M, Alegre M, Alonso-Usero V, Boixeda P, Dominguez-Silva J, Fernández-Herrera J, García-Navarro X, Jiménez N, Llamas M, Nadal C, Del Pozo-Losada J, Querol I, Salgüero I, Schaller M, Soto de Delás J. Spanish Consensus Document on the Treatment Algorithm for Rosacea. Actas Dermosifiliogr (Engl Ed). 2019 Sep;110(7):533-545. English, Spanish. doi: 10.1016/j.ad.2019.01.001. Epub 2019 Mar 2. PMID: 30837074.
Sánchez-Pellicer P, Eguren-Michelena C, García-Gavín J, Llamas-Velasco M, Navarro-Moratalla L, Núñez-Delegido E, Agüera-Santos J, Navarro-López V. Rosacea, microbiome and probiotics: the gut-skin axis. Front Microbiol. 2024 Jan 8;14:1323644. doi: 10.3389/fmicb.2023.1323644. PMID: 38260914; PMCID: PMC10800857.
Sánchez-Pellicer P, Navarro-Moratalla L, Núñez-Delegido E, Ruzafa-Costas B, Agüera-Santos J, Navarro-López V. Acne, Microbiome, and Probiotics: The Gut-Skin Axis. Microorganisms. 2022 Jun 27;10(7):1303. doi: 10.3390/microorganisms10071303. PMID: 35889022; PMCID: PMC9318165.
Schaller M, Almeida LMC, Bewley A, Cribier B, Del Rosso J, Dlova NC, Gallo RL, Granstein RD, Kautz G, Mannis MJ, Micali G, Oon HH, Rajagopalan M, Steinhoff M, Tanghetti E, Thiboutot D, Troielli P, Webster G, Zierhut M, van Zuuren EJ, Tan J. Recommendations for rosacea diagnosis, classification and management: update from the global ROSacea COnsensus 2019 panel. Br J Dermatol. 2020 May;182(5):1269-1276. doi: 10.1111/bjd.18420. Epub 2019 Oct 16. PMID: 31392722; PMCID: PMC7317217.
Schaller M, Almeida LM, Bewley A, Cribier B, Dlova NC, Kautz G, Mannis M, Oon HH, Rajagopalan M, Steinhoff M, Thiboutot D, Troielli P, Webster G, Wu Y, van Zuuren E, Tan J. Rosacea treatment update: recommendations from the global ROSacea COnsensus (ROSCO) panel. Br J Dermatol. 2017 Feb;176(2):465-471. doi: 10.1111/bjd.15173. Epub 2017 Feb 5. PMID: 27861741.
Thoda C, Touraki, M. Immunomodulatory Properties of Probiotics and Their Derived Bioactive Compounds. Appl. Sci. 2023, 13, 4726. https://doi.org/10.3390/app13084726
van Zuuren EJ. Rosacea. N Engl J Med. 2017 Nov 2;377(18):1754-1764. doi: 10.1056/NEJMcp1506630. PMID: 29091565.

## Claims

1. A probiotic composition comprising:
a. *L. rhamnosus* strain deposited under the Budapest treaty in the Spanish Type culture Collection (CECT) under accession number CECT 30579; and
b. *B. longum* strain deposited under the Budapest treaty in the Spanish Type culture Collection (CECT) under accession number CECT 30615.

2. The probiotic composition of claim 1, wherein the composition comprises between 10⁶ and 10¹² cfu, preferably between 10⁹ and 10¹¹ cfu of *L. rhamnosus* CECT 30579, and between 10⁶ and 10¹² cfu, preferably between 10⁹ and 10¹¹ cfu of *B. longum* CECT 30615.

3. The probiotic composition according to claims 1 or 2, wherein said composition is in the form of an air dried or lyophilized (freeze-dried) powder.

4. The probiotic composition according to claims 1 to 3, wherein said composition is formulated for administration in liquid form or in solid form, preferably wherein said composition is formulated for oral administration.

5. The probiotic composition according to any of claims 1 to 4, wherein said composition is a pharmaceutical composition comprising pharmaceutically acceptable excipients.

6. The probiotic composition according to any of claims 1 to 5, wherein said composition is a nutritional composition or nutritional supplement.

7. The nutritional composition or nutritional supplement according to claim 6 or the pharmaceutical composition according to claim 5, wherein said composition is in the form of a formulation selected from the group consisting of tablets, lozenges, sweets, chewable tablets, chewing gum, capsules, sachets, powders, granules, coated particles or coated tablets, tablets and gastro-resistant tablets and capsules and dispersible strips and films, or in the form of a liquid formulation selected from the group consisting of oral solutions, suspensions, emulsions and syrups.

8. The composition according to any of claims 1 to 7, for use in therapy.

9. The probiotic composition according to any of claims 1 to 8, for use in a method of easing, reducing, treating and/or preventing Rosacea in a subject, preferably a human subject, in need thereof.

10. The probiotic composition according to claim 9, for use in the method of treating Rosacea in a subject, preferably a human subject in need thereof, further comprising orally administering to said subject an effective amount of the composition thereby treating said Rosacea.

11. The probiotic composition, for use in the method of treating Rosacea in a subject, preferably a human subject, in need thereof according to any of claims 9 or 10, wherein at least one additional compound is administered in an effective amount for treating Rosacea to said subject, preferably wherein said additional compound acts as an adjuvant.

12. A probiotic composition comprising *L. rhamnosus* strain deposited under the Budapest treaty in the Spanish Type culture Collection (CECT) under accession number CECT 30579, for use in a method of easing, reducing, treating and/or preventing Rosacea in a subject, preferably a human subject, in need thereof.

13. A probiotic composition comprising *B. longum* strain deposited under the Budapest treaty in the Spanish Type culture Collection (CECT) under accession number CECT 30615 for use in a method of easing, reducing, treating and/or preventing Rosacea in a subject, preferably a human subject, in need thereof.

14. The probiotic composition for use according to claims 12 or 13, wherein the composition comprises at least between 10⁶ and 10¹² cfu, preferably between 10⁹ and 10¹¹ cfu of *L. rhamnosus* CECT 30579 and between 10⁶ and 10¹² cfu, preferably between 10⁹ and 10¹¹ of *B. longum* CECT 30615.

15. The probiotic composition for use according to any one of claims 12 to 14, wherein the probiotic composition is administered orally to the subject in need thereof.
